# EUROPÄISCHE PATENTANMELDUNG

(11) **EP 1 938 783 A2**
(43) Veröffentlichungstag der Anmeldung: **02.07.2008**
(21) Anmeldenummer: 07023933.0
(22) Anmeldetag: 11.12.2007
(51) Int. Cl.: A61K 8/04, A61K 8/34, A61Q 15/00

(54) **Deodorant-Emulsionsspray**

(30) Priorität: 28.12.2006 DE 102006062499
(71) Anmelder: Henkel AG & Co. KGaA, 40589 Düsseldorf (DE)
(72) Erfinder: Kaftan, Pamela, 22761 Hamburg (DE); Emmerling, Winfried, 25436 Tornesch (DE)

(57) **Zusammenfassung**

Die Erfindung betrifft ein deodorierendes Körperpflegemittel in Form einer Wasser-in-Öl-Emulsion, die mindestens 10 Gew.-% Ethanol enthält und als Aerosolspray konfektioniert ist.

## Beschreibung

Die Erfindung betrifft ein deodorierendes Körperpflegemittel in Form einer Wasser-in-Öl-Emulsion, die mindestens 10 Gew.-% Ethanol enthält und als Aerosolspray konfektioniert ist.

Ein Aerosol ist ein disperses System, bei dem ein Feststoff oder eine Flüssigkeit in einem Gas sehr fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel-Zubereitungsgemisch durch eine feine Düse, das Treibmittel verdunstet und hinterlässt das fein verteilte Sprühgut als Aerosol.

Aerosol-Deodorantsprays erfreuen sich im Körperpflegebereich zunehmender Beliebtheit. Gängige Deodorantsprayzusammensetzungen liegen als wasserfreie ethanolische Lösungen des deodorierenden Wirkstoffes neben dem Treibgas vor. Ein Nachteil dieser ethanolischen Lösungen ist bei entsprechend prädisponierten Personen mit empfindlicher Haut eine hautreizende Wirkung. Auch auf durch eine Rasur mechanisch gereizter Haut führt die Applikation der gängigen ethanolischen Lösungen zu einem sehr unangenehmen Brennen.

Ein weiterer Nachteil ethanolischer Lösungen ist, dass die Einarbeitung wasserhaltiger oder wasserlöslicher Wirkstoffe, insbesondere von Deodorant-Wirkstoffen oder haarwuchshemmenden Wirkstoffen, die nicht auch in Ethanol löslich sind, nicht oder nur sehr eingeschränkt möglich ist.

Aufgabe der vorliegenden Erfindung war es, ein Deodorant-Produkt auf Basis eines Aerosolsprays mit Deodorant-Wirkstoffen mit einer Abgabevorrichtung zu entwickeln, das eine in Bezug auf die Hautverträglichkeit verbesserte Produktleistung aufweist. Eine weitere Aufgabe war es, ein Deodorant-Produkt auf Basis eines Aerosolsprays mit Deodorant-Wirkstoffen bereitzustellen, das problemlos mit wasser-, alkohol- oder glycol-haltigen Rohstoffen, z. B. Extrakten oder Wirkstofflösungen, formuliert werden kann. Eine weitere Aufgabe war es, ein Deodorant-Produkt auf Basis einer wasserhaltigen Emulsion mit Deodorant-Wirkstoffen mit einer Abgabevorrichtung zu entwickeln, das verringerte Korrosionseigenschaften aufweist. Eine weitere Aufgabe war es, ein Deodorant-Produkt auf Basis einer wasserhaltigen Emulsion mit Deodorant-Wirkstoffen mit einer Abgabevorrichtung zu entwickeln, das ein verbessertes Frischegefühl auf der Haut aufweist.
Für eine besonders effiziente Anwendung haarwuchshemmender Wirkstoffe ist es insbesondere sinnvoll, wenn die Haut vor der Produktapplikation rasiert wurde. Dabei kommt es aber zu Hautreizungen, was es erforderlich und dem Fachmann zur Aufgabe macht, dass das anschließend aufgetragene kosmetische Produkt möglichst mild und reizarm ist.

Überraschend wurde nun gefunden, dass die bestehenden Probleme überwunden werden durch ein Deodorant-Aerosolspray auf Basis einer Wasser-in-Öl-Emulsion mit mindestens 10 Gew.-% Ethanol, bezogen auf das Gewicht der treibgasfreien Emulsion.

Ein Gegenstand der vorliegenden Erfindung ist daher ein deodorierendes kosmetisches Produkt, umfassend
- eine Wasser-in-Öl-Emulsion mit mindestens einem Deodorant-Wirkstoff,
- mindestens ein Treibmittel und
- eine Aerosol-Abgabevorrichtung,
wobei mindestens 10 Gew.-% Ethanol, bezogen auf das Gewicht der treibgasfreien Emulsion, enthalten sind.

Das erfindungsgemäße deodorierende kosmetische Produkt umfasst eine Wasser-in-Öl-Emulsion mit einem Ethanolgehalt von mindestens 10 Gew.-%, bezogen auf das Gewicht der treibgasfreien Emulsion. Die Wasser-in-Öl-Emulsion des erfindungsgemäßen deodorierenden Produktes umfasst ihrerseits eine Ölphase, wobei bevorzugte erfindungsgemäße kosmetische Produkte dadurch gekennzeichnet sind, dass die Wasser-in-Öl-Emulsion eine Ölphase von 1 - 60 Gew.-%, bevorzugt 5 - 30 Gew.-%, besonders bevorzugt 22 - 26 Gew.-%, jeweils bezogen auf das Gesamtgewicht der Emulsion, enthält. Die Emulgatoren werden erfindungsgemäß weder zur Ölphase noch zur Wasserphase gezählt.

Weiterhin besteht die Ölphase vorzugsweise zu mindestens 90 Gew.-% aus bei 20 °C flüssigen Ölkomponenten. Geeignete Ölkomponenten sind ausgewählt aus:
- flüchtigen und nichtflüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind, sowie nichtflüchtige höhermolekulare lineare Dimethylpolysiloxane, im Handel erhältlich z. B. unter der Bezeichnung Dow Corning^{®} 200 Fluid mit Viskositäten im Bereich von 5 - 100 cSt, bevorzugt 5 - 50 cSt oder auch 5 - 10 cSt, und Baysilon^{®} 350 M;
- den Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen bevorzugt 2-Ethylhexylpalmitat (z. B. Cegesoft^{®} C 24), Hexyldecylstearat (Eutanol^{®} G 16), Hexyldecyllaurat, Isodecylneopentanoat, Isononylisononanoat, 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanot, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat;
- den Benzoesäureestern von linearen oder verzweigten C₈₋₂₂-Alkanolen, z. B. die Handelsprodukte Finsolv^{®} TN (C₁₂-C₁₅-Alkylbenzoat), Finsolv® SB (Isostearylbenzoat) und Finsolv® EB (Ethylhexylbenzoat);
- den C₈-C₂₂-Fettalkoholestern einwertiger oder mehrwertiger C₂-C₇-Hydroxycarbonsäuren, insbesondere den Estern der Glycolsäure, Milchsäure, Äpfelsäure, Weinsäure, Citronensäure und Salicylsäure. Solche Ester auf Basis von linearen C_{12/15}-Alkanolen, z. B. C₁₂-C₁₅-Alkyllactat, und von in 2-Position verzweigten C_{12/13}-Alkanolen, z. B. Di-C₁₂-C₁₃-Alkylmalat, sind unter dem Warenzeichen Cosmacol® von der Firma Nordmann, Rassmann GmbH & Co, Hamburg, zu beziehen, insbesondere die Handelsprodukte Cosmacol® EMI, Cosmacol® ESI und Cosmacol® ETI;
- den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57), PPG-3-Myristylether (Witconol^{®} APM) und PPG-15-Stearylether (Arlamol^{®} E);
- flüssigen Paraffinölen, Isoparaffinölen, z. B. die Handelsprodukte der Permethyl^{®}-Serie, insbesondere Isododecan, Isohexadecan und Isoeicosan, und synthetischen Kohlenwasserstoffen wie Polyisobuten oder Polydecene und alicyclischen Kohlenwasserstoffen, z. B. das Handelsprodukt 1,3-Di-(2-ethyl-hexyl)-cyclohexan (Cetiol^{®} S);
- den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Besonders bevorzugte Alkoholöle sind beispielsweise Hexyldecanol (Eutanol^{®} G), Octyldodecanol und 2-Ethylhexylalkohol;
- Mischungen aus Guerbetalkoholen und Guerbetalkoholestern, z. B. das Handelsprodukt Cetiol^{®} PGL (Hexyldecanol und Hexyldecyllaurat).
- den symmetrischen, unsymmetrischen oder cyclischen Estern der Kohlensäure mit Fettalkoholen, beispielsweise Glycerincarbonat, Dicaprylylcarbonat (Cetiol^{®} CC) oder die Ester der DE-OS 197 56 454;
- Triglyceriden von linearen oder verzweigten, gesättigten oder ungesättigten, gegebenenfalls hydroxylierten C₈₋₃₀-Fettsäuren. Geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Besonders bevorzugt sind aber die synthetischen Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten;
- Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat;
- Di-n-alkylethern mit insgesamt 12 bis 36, insbesondere 12 bis 24 C-Atomen, z. B. Di-n-octylether (Cetiol^{®} OE), Di-n-decylether, n-Hexyl-n-octylether und n-Octyl-n-decylether.

Besonders bevorzugte Öle sind die flüchtigen cyclischen Siliconöle Decamethylcyclopentasiloxan und Dodecamethylcyclohexasiloxan, die flüchtigen linearen Siliconöle Hexamethyldisiloxan (L₂), Octamethyltrisiloxan (L₃) und Decamethyltetrasiloxan (L₄) sowie beliebige Mischungen, insbesondere Zweier- und Dreiermischungen, aus L₂, L₃ und/oder L₄, flüchtige und nichtflüchtige lineare Siliconöle aus der Serie Dow Corning 200 Fluid mit Viskositäten von 0,65, 1,0, 1,5 und 5 cSt, die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat, Di-C₁₂-C₁₃-Alkylmalat, PPG-14-Butylether (Ucon Fluid^{®} AP), die Handelsprodukte der Permethyl^{®}-Serie, insbesondere Isododecan, Isohexadecan und Isoeicosan, sowie Polyisobuten und Polydecene sowie Mischungen der genannten Komponenten. Besonders bevorzugte erfindungsgemäße kosmetische Produkte sind dadurch gekennzeichnet, dass die Ölkomponenten ausgewählt sind aus Decamethylcyclopentasiloxan, Dodecamethylcyclohexasiloxan, Hexamethyldisiloxan (L₂), Octamethyltrisiloxan (L₃), Decamethyltetrasiloxan (L₄), beliebigen Mischungen, insbesondere Zweier- und Dreiermischungen, aus L₂, L₃ und/ oder L₄, weiterhin 2-Ethylhexylpalmitat, Hexyldecyllaurat, 2-Ethylhexylstearat, 2-Ethylhexyllaurat, Isopropylmyristat, Isopropylpalmitat, C₁₂-C₁₅-Alkylbenzoat, C₁₂-C₁₅-Alkyllactat, Di-C₁₂-C₁₃-Alkylmalat, PPG-14-Butylether, Isododecan, Isohexadecan, Isoeicosan, Polyisobuten und Polydecene sowie Mischungen der genannten Komponenten.
Es kann erfindungsgemäß bevorzugt sein, Mischungen der vorgenannten Öle einzusetzen. Dabei sind besonders Mischungen aus zwei Ölkomponententypen, z. B. flüchtiges Siliconöl und Esteröl, bevorzugt. Besonders bevorzugt sind Ölmischungen, die mindestens ein flüchtiges cyclisches und/oder lineares Siliconöl enthalten. Außerordentlich bevorzugt sind Ölmischungen, die überwiegend, das heißt zu mehr als 50 Gew.-%, mindestens ein flüchtiges cyclisches und/oder lineares Siliconöl enthalten. Entsprechende kosmetische Produkte, bei denen die Ölphase eine Ölmischung, die zu mehr als 50 Gew.-% mindestens ein flüchtiges cyclisches oder lineares Siliconöl enthält, umfasst, sind besonders bevorzugt.

Weiter bevorzugt sind auch erfindungsgemäße kosmetische Produkte, bei denen die Ölphase zu mindestens 90 Gew.-% aus bei 20 °C flüssigen Ölkomponenten besteht sowie kosmetische Produkte, bei denen mindestens 80 Gew.-% der Ölkomponenten einen Brechungsindex n_{D} von 1,39 - 1,49 bei 20 °C (gemessen bei λ = 589 nm) aufweisen.

Die Wasser-in-Öl-Emulsion des erfindungsgemäßen deodorierenden Produktes enthält Wasser, wobei dies vorzugsweise 30 - 60 Gew.-%, besonders bevorzugt 40 - 50 Gew.-% des Gesamtgewichts der treibgasfreien Emulsion ausmacht. Die Emulgatoren sowie der Ethanolgehalt werden erfindungsgemäß weder zur Wasserphase noch zur Ölphase gezählt.
Zur Wasserphase zählen erfindungsgemäß Wasser sowie alle wasserlöslichen Inhaltsstoffe, mit Ausnahme der Emulgatoren und des Ethanols.

Die Wasser-in-Öl-Emulsion des erfindungsgemäßen deodorierenden Produktes enthält weiterhin mindestens einen Wasser-in-Öl-Emulgator. Kosmetische Produkte, bei denen die Wasser-in-Öl-Emulsion mindestens einen Wasser-in-Öl-Emulgator in einer Gesamtmenge von 0,1 - 10 Gew.-%, bevorzugt 0,5 - 5 Gew.-%, besonders bevorzugt 1 - 3 Gew.-%, jeweils bezogen auf das Gesamtgewicht der treibgasfreien Emulsion, enthält, sind erfindungsgemäß bevorzugt.

Eine erfindungsgemäß besonders bevorzugte Gruppe von Wasser-in-Öl-Emulgatoren sind die Poly-(C₂-C₃)alkylenglycol-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in unverdünnter Form z.B. unter der Handelsbezeichnung DC 190 oder in einer 1:9-Mischung mit Cyclomethicone als DC 3225 C bzw. DC 5225 C im Handel erhältlich ist, sowie Bis-PEG/PPG-14/14 Dimethicone, das in einer Mischung mit Cyclomethicone als Abil EM 97 (Goldschmidt) im Handel erhältlich ist.

Weitere erfindungsgemäß außerordentlich bevorzugte W/O-Emulgatoren sind Poly-(C₂-C₃)alkylenglycol-modifizierte Silicone, die mit C₄-C₁₈-Alkylgruppen hydrophob modifiziert sind. Besonders bevorzugte Silicon-W/O-Emulgatoren sind Cetyl PEG/PPG-10/1 Dimethicone (früher: Cetyl Dimethicone Copolyol, erhältlich als Abil EM 90), Lauryl Dimethicone Copolyol, Lauryl Dimethicone PEG-15 Crosspolymer, PEG-10/Lauryl Dimethicone Crosspolymer, PEG-8 Cetyl Dimethicone, weiterhin Alkyl Methicone Copolyole und Alkyl Dimethicone Ethoxy Glucoside.
Erfindungsgemäß bevorzugte Produkte enthalten mindestens einen Silicon-W/O-Emulgator in einer Gesamtmenge von 0,1 - 10 Gew.-%, bevorzugt 0,5 - 5 Gew.-%, besonders bevorzugt 1 - 3 Gew.-%, jeweils bezogen auf das Gesamtgewicht der treibgasfreien Emulsion.
Erfindungsgemäß besonders bevorzugte Produkte enthalten als einzigen W/O-Emulgator 1 - 3 Gew.-% Cetyl PEG/PPG-10/1 Dimethicone, bezogen auf das Gewicht der treibgasfreien W/O-Emulsion.
Weitere erfindungsgemäß besonders bevorzugte Produkte enthalten als einzigen W/O-Emulgator 1
- 3 Gew.-% Lauryl Dimethicone PEG-15 Crosspolymer, bezogen auf das Gewicht der treibgasfreien W/O-Emulsion.
   Weitere erfindungsgemäß besonders bevorzugte Produkte enthalten als einzigen W/O-Emulgator 1
- 3 Gew.-% Lauryl Dimethicone Copolyol, bezogen auf das Gewicht der treibgasfreien W/O-Emulsion.

Weitere erfindungsgemäß geeignete W/O-Emulgatoren sind ausgewählt aus Substanzen der allgemeinen Formel A-O-(CHR¹-X-CHR²-O-)ₐ-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe >CHOR³ darstellt, R¹ und R² ein Wasserstoffatom oder eine Methylgruppe darstellen und so gewählt werden, dass nicht beide Reste gleichzeitig Methyl darstellen, und R³ ein Wasserstoffatom oder eine verzweigte oder unverzweigte, gesättigte oder ungesättigte Alkyl- oder Acylgruppe mit 1 bis 20 Kohlenstoffatomen darstellt.
Besonders bevorzugt ist es, wenn der W/O-Emulgator oder die W/O-Emulgatoren so gewählt werden, dass die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H)_{b}-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.
Weitere bevorzugte W/O-Emulgatoren sind ausgewählt aus
(1) gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden;
(2) ethoxylierten Alkoholen und Carbonsäuren mit 8 - 24 C-Atomen, insbesondere mit 16 - 22 C-Atomen, die einen HLB-Wert von 1 - 8 aufweisen;
(3) propoxylierten Alkoholen und Carbonsäuren mit 8 - 24 C-Atomen, insbesondere mit 16 - 22 C-Atomen;
(4) Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar.
(5) Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Veresterungsgrad von 1 - 10, vorzugsweise 1 - 5;
(6) Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1 -10, vorzugsweise 1 - 5;
(7) Propylenglycolestern gesättigter und/oder ungesättigter, verzweigter und/oder unverzweigter Alkancarbonsäuren einer Kettenlänge von 8 - 24, insbesondere 12 -18 C-Atomen;
(8) Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(9) Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen.
   Es kann erfindungsgemäß von Vorteil sein, dass niedrig ethoxylierte (3 - 5 EO) und/oder propoxylierte Produkte Verwendung finden, beispielsweise polyethoxyliertes hydriertes oder nichthydriertes Ricinusöl oder ethoxyliertes Cholesterin.

Besonders bevorzugte W/O-Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearyl-ether (Steareth-2), Glycerylsorbitanstearat, Polyglyceryl-4-Isostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyceryl-3-diisostearat, PEG-8 Distearat, Diglycerin Dipolyhydroxystearat, Glycerinisostearat, Sorbitanisostearat, Polyglyceryl-3-methylglucosedistearat, polyethoxyliertes hydriertes oder nichthydriertes Ricinusöl, ethoxyliertes Cholesterin, PEG-2 Stearat, PEG-45/Dodecylglycolcopolymer, PEG-22/Dodecylglycolcopolymer und Methoxy PEG-22/Dodecyl Glycol Copolymer.
Ganz besonders bevorzugt kann es sein, wenn Kombinationen der oben genannten siliconfreien W/O-Emulgatoren, insbesondere eine Kombination aus zwei siliconfreien W/O-Emulgatoren, eingesetzt werden. Ein besonders bevorzugter siliconfreier W/O-Emulgator ist PEG-30 Dipolyhydroxystearat, erhältlich von Uniqema unter dem Handelsnamen Arlacel P 135.
Es kann erfindungsgemäß vorteilhaft sein, zusätzlich zu dem mindestens einen W/O-Emulgator auch mindestens einen O/W-Emulgator einzusetzen.

Die W/O-Emulsion des erfindungsgemäßen deodorierenden Produktes enthält mindestens 10 Gew.-% Ethanol, bezogen auf das Gewicht der treibgasfreien W/O-Emulsion. Besonders bevorzugt ist ein Ethanolgehalt von 15 - 50 Gew.-%, außerordentlich bevorzugt 24 - 40 Gew.-% Ethanol. Überraschend wurde festgestellt, dass die Konfektionierung eines mittleren bis höheren Ethanolgehalts als W/O-Emulsion das Ethanol wesentlich hautverträglicher macht. Dies wurde insbesondere beobachtet bei W/O-Emulsionen, die als einzigen W/O-Emulgator Cetyl PEG/PPG-10/1 Dimethicone oder Lauryl Dimethicone Copolyol oder Lauryl Dimethicone PEG-15 Crosspolymer oder PEG-10/Lauryl Dimethicone Crosspolymer, enthalten. Speziell mit Cetyl PEG/PPG-10/1 Dimethicone wurde auch überraschend beobachtet, dass der Frischeeffekt, der durch den Ethanolgehalt der erfindungsgemäßen W/O-Emulsionen hervorgerufen wird, dem der herkömmlichen ethanolischen Lösungen entspricht beziehungsweise diesen sogar übertrifft, und zwar trotz des höheren Wassergehaltes.

In einer weiteren bevorzugten Ausführungsform enthalten die W/O-Emulsionen des erfindungsgemäßen Produktes mindestens ein wasserlösliches Polyol, ausgewählt aus den wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten sowie Mischungen hiervon. Besonders bevorzugte erfindungsgemäße kosmetische Produkte sind dadurch gekennzeichnet, dass mindestens ein wasserlösliches mehrwertiges C₂ - C₉-Alkanol mit 2 - 6 Hydroxylgruppen und/oder wasserlösliches Polyethylenglycol mit 3 - 20 Ethylenoxid-Einheiten in einer Gesamtmenge von 0,5 - 15 Gew.-%, bevorzugt 4 - 10 Gew.-% und besonders bevorzugt 5 - 7 Gew.-%, jeweils bezogen auf die gesamte treibgasfreie W/O-Emulsion, enthalten ist.

Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen, Hexandiolen wie 1,2 Hexandiol, 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose können erfindungsgemäß geeignet sein. Besonders bevorzugt sind 1,2-Propylenglycol und 1,3-Butylenglycol.

Die W/O-Emulsionen des erfindungsgemäßen Produktes können weiterhin bevorzugt ein oder mehrere Konservierungsmittel enthalten. Erfindungsgemäß bevorzugte Konservierungsmittel sind Formaldehydabspalter (wie z. B. 1,3-Dimethylol-4,4-dimethylhydantoin, INCI-Bezeichnung DMDM Hydantoin, z. B. unter der Handelsbezeichnung Glydant von der Firma Lonza erhältlich), lodopropinylbutylcarbamate wie 3-lod-2-propinylbutylcarbamat (z. B. die unter den Handelsbezeichnungen Glycacil-L, Glycacil-S von der Firma Lonza erhältlichen und/oder Dekaben LMB von Jan Dekker), Parabene (d. h. p- Hydroxybenzoesäurealkylester, wie Methyl-, Ethyl-, Propyl- und/oder Butylparaben), Phenoxyethanol, Ethanol, Benzoesäure, Dibromdicyanobutan (2-Brom-2-brommethylglutarodinitril), 2-Brom-2-nitro-propan-1,3-diol, Imidazolidinylharnstoff, 5-Chlor-2-methyl-4-isothiazolin-3-on, 2- Chloracetamid, Benzalkoniumchlorid, Benzylalkohol, Salicylsäure und Salicylate.
Erfindungsgemäß besonders bevorzugte Konservierungsmittel sind ausgewählt aus lodopropinylbutylcarbamaten, Parabenen (Methyl-, Ethyl-, Propyl- und/oder Butylparaben) und/ oder Phenoxyethanol.
Die Konservierungsmittel sind vorzugsweise in Mengen von 0,01 - 2, bevorzugt 0,1 - 1,5 und besonders bevorzugt 0,2 - 1,0 Gew.-% enthalten, jeweils bezogen auf das Gewicht der W/O-Emulsion.

Die erfindungsgemäßen W/O-Emulsionen können bevorzugt weiterhin mindestens eine Duftstoffkomponente enthalten. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Phenoxyethylisobutyrat, Benzylacetat, p-tert.-Butylcyclohexylacetat, Dimethylbenzylcarbinylacetat, Linalylacetat, Phenylethylacetat, Linalylbenzoat, Ethylmethylphenylglycinat, Benzylformiat, Allylcyclohexylpropionat, Styrallylpropionat und Benzylsalicylat. Zu den Ethern zählen zum Beispiel Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die lonone α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Geeignete Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl.
Die Duftstoffkomponente/n ist/sind bevorzugt in Mengen von 0,01 bis 4 Gew.-%, besonders bevorzugt 0,5 - 2 Gew.-%, jeweils bezogen auf das Gewicht der treibgasfreien W/O-Emulsion, enthalten.

Die W/O-Emulsionen des erfindungsgemäßen Produktes können vorteilhafterweise weiterhin mindestens einen hautkühlenden Wirkstoff enthalten. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat und 2- Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.
Die erfindungsgemäß verwendeten W/O-Emulsionen enthalten vorzugsweise mindestens einen hautkühlenden Wirkstoff in Mengen von 0,01 - 1 Gew.%, bevorzugt 0,02 - 0,5 Gew.% und besonders bevorzugt 0,05 - 0,2 Gew.%, jeweils bezogen auf das Gesamtgewicht der W/O-Emulsion.

Die erfindungsgemäß verwendeten W/O-Emulsionen können bevorzugt weiterhin mindestens einen Pflanzenextrakt enthalten. Pflanzenextrakte werden üblicherweise durch Extraktion der gesamten Pflanze, in einzelnen Fällen aber auch ausschließlich aus Blüten und/oder Blättern und/oder Samen und/oder anderen Pflanzenteilen, hergestellt. Erfindungsgemäß bevorzugt sind vor allem die Extrakte aus Aloe Vera, Grünem Tee, Hamamelis, Bambus, Kamille, Ringelblume, Stiefmütterchen, Paeonie, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel und Süßkartoffel. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesiumangereicherten Medium kultiviert wurden.
Die erfindungsgemäßen W/O-Emulsionen können auch Mischungen aus mehreren, insbesondere aus zwei, verschiedenen Pflanzenextrakten enthalten.
Diese erfindungsgemäßen W/O-Emulsionen enthalten vorzugsweise mindestens einen Pflanzenextrakt in Mengen von 0,01 - 5 Gew.%, bevorzugt 0,1 - 2 Gew.% und besonders bevorzugt 0,5 - 1,0 Gew.%, jeweils bezogen auf das Gesamtgewicht der W/O-Emulsion.

In einer weiteren bevorzugten Ausführungsform enthalten die erfindungsgemäß verwendeten W/O-Emulsionen mindestens ein Vitamin, Provitamin oder eine als Vitaminvorstufe bezeichnete Verbindung aus den Vitamingruppen A, B, C, E, H und K und den Estern der vorgenannten Substanzen.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäß verwendeten W/O-Emulsionen enthalten die Vitamin A-Komponente bevorzugt in Mengen von 0,05 - 1 Gew.-%, bezogen auf die gesamte W/O-Emulsion. Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte W/O-Emulsion, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte W/O-Emulsion, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte W/O-Emulsion, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden. 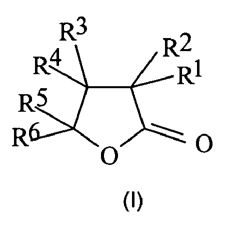 Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
   Die genannten Verbindungen des Vitamin B₅-Typs sowie die 2-Furanonderivate sind in den erfindungsgemäßen Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte W/O-Emulsion, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere 0,001 bis 0,01 Gew.-%, jeweils bezogen auf die gesamte W/O-Emulsion, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere 0,001 bis 0,01 Gew.-%, jeweils bezogen auf die gesamte W/O-Emulsion, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die gesamte treibgasfreie W/O-Emulsion, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die gesamte treibgasfreie W/O-Emulsion, enthalten.
   Vitamin C (Ascorbinsäure) wird bevorzugt in Mengen von 0,1 bis 3 Gew.-%, bezogen auf die gesamte W/O-Emulsion, eingesetzt. Die Verwendung der Derivate Ascorbylpalmitat, -stearat, - dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
   Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte treibgasfreie W/O-Emulsion, enthalten.

Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte treibgasfreie W/O-Emulsion, enthalten.
Vitamin A-palmitat (Retinylpalmitat), Panthenol, Pantolacton, Nicotinsäureamid, Pyridoxin, Pyridoxamin, Pyridoxal, Biotin, Folsäure, Ascorbylpalmitat, -acetat, Mg-Ascorbylphosphat, Natriumascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen W/O-Emulsionen mindestens einen hautberuhigenden Wirkstoff. Erfindungsgemäß bevorzugte hautberuhigende Wirkstoffe sind ausgewählt aus Allantoin, α-Bisabolol, α-Liponsäure und (2-Hydroxyethyl)harnstoff.
Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte W/O-Emulsion, enthalten.

Bevorzugte erfindungsgemäße deodorierende Produkte enthalten in der Wasser-in-Öl-Emulsion mindestens einen Wirkstoff mit haarwuchshemmender Wirkung, also eine den Haarwuchs inhibierende Substanz. Geeignete Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Sérobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (pumpkin, Zucchini) und den Früchten von Serenoa serrulata, wie sie beispielsweise und bevorzugt in den Rohstoffen ARP 100 von Greentech S.A./Rahn mit der INCI-Deklaration "Water, Alcohol, Serenoa Serrulata Fruit Extract, Epilobium Angustifolium Extract, Cucurbita Pepo (Pumpkin) Seed Extract" und ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag / Seporga mit der INCI-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind.
Weitere bevorzugte Haarwuchs-inhibierende Wirkstoffe sind ausgewählt aus Substanzen, die die Protein-Tyrosinkinase hemmen, insbesondere aus Lavendustin-A, Erbstatin, Tyrphostin, Piceatannol, 4-Hydroxybenxylidenmalononitril, 3,5-Di-*tert*-butyl-4-hydroxybenzylidenmalononitril, a-Cyano-(3,4-dihydroxy)-cinnamonitril, α-Cyano-(3,4,5-trihydroxy)cinnamonitril, α-Cyano-(3,4-dihydroxy)-cinnamid, α-Cyano-(3,4-dihydroxy)thiocinnamid, 2- Amino-4-(4'-hydroxyphenyl)-1,1,3-tricyanobuta-1,3-dien, 2-Amino-4-(3,4,5'- trihydroxyphenyl)-1,1,3-tricyanobuta-1,3-dien, 2-Amino-4-(1H-alpha-indol-5-yl)-1,1,3-tricyanobuta-1,3-dien, 4-Hydroxy-3-methoxy-5-(benzothiazolylthiomethyl)benzylidencyanoacetamid, 4-Amino-N-(2,5-dihydroxybenzyl)methylbenzoat, α-Cyano-(3,4-dihydroxy)-cinnamonitril, 4-(3-Chloranilino)-6,7-dimethoxychinazolin, α-Cyano-(3,4-dihydroxy)-N-benzylcinnamid, (-)-R-N-(a-Methylbenzyl)-3,4-dihydroxybenzylidencyanoacetamid, α-Cyano-(3,4-dihydroxy)-N-(3-phenylpropyl)-cinnamid, α-Cyano-(3,4-dihydroxy)-N-phenylcinnamid, α-Cyano-(+)-(S)-N-(alpha-phenethyl)-(3,4-dihydroxy)cinnamid, α-Cyano-(3,4-dihydroxy)-N-(phenylbutyl)cinnamid, Herbimycin A, Thiazolidindion, Phenazocin, 2,3-Dihydro-2-thioxo-1H-indol-3-Alkansäuren, 2,2'-Dithiobis-(1H-indol-3-Alkansäuren), Sulfonylbenzoylnitrostyrol, Methylcaffeat, HNMPA(AM)₃(hydroxy-2-naphthalenylmethylphosphonsäure-*tris*-acetoxymethylester) und N-Acetyl-Asp-Tyr-(2-malonyl)-Val-Pro-Met-Leu-NH₂.
Weitere bevorzugte Haarwuchs-inhibierende Wirkstoffe sind ausgewählt aus den Substanzen, die in WO 2006/130330 A2 offenbart sind, nämlich Agonisten des Farnesoid X-Rezeptors, bevorzugt ausgewählt aus Gallensäuren, wie insbesondere Lithocholsäure, Cholsäure, Deoxycholsäure, Chenodeoxycholsäure, Ursodeoxycholsäure und 6-alpha-Ethylchenodeoxycholsäure, weiterhin aus Farnesoiden, insbesondere Farnesol (3,7,11-Trimethyl-2,6,10-dodecatrien-1-ol), Farnesal, Farnesylacetat, 3,7,11-Trimethyl-2,6,10-dodecatrien-1-carbonsäure, Methylfarnesylether, Methylfarnesoat, Ethylfarnesylether, Ethylfarnesoat, weiterhin aus 7-Methyl-9-(3,3-dimethyloxivanyl)-3-methyl-2,6-nonadiensäuremethylester (Juvenilhormon III), 7-Methyl-9-(3,3-dimethyloxivanyl)-3-methyl-2,6-nonadiensäureethylester, 3-alpha,7-alpha-Dihydroxy-6-alpha-ethyl-5p-cholan-24-säure, 7-alpha-Dihydroxy-6-alpha-propyl-5p-cholan-24-säure, 7-alpha-Dihydroxy-6-alpha-allyl-5p-cholan-24-säure, N-(2,2,2-trifluoroethyl)-N-[4-[2,2,2-trifluoro-1-hydroxy-1-(trifluoromethyl)-ethyl]phenyl]-benzolsulfonanilid, 3-[2-[2-Chloro-4-[3-(2,6-dichlorophenyl)-5-(1-methylethyl)-4-isoxazolyl]-methoxy]-phenylethenyl]-benzoesäure, [3,5-bis(1,1-dimethylethyl)-4-hydroxyphenyl]ethenyliden]-bisphosphonsäuretetraethylester, [2-[3,5-bis(1,1-dimethylethyl)-4-hydroxyphenyl]ethyliden]bisphosphonsäuretetrakis(1-methylethyl)ester, [2-[3,5-bis(1,1-dimethylethyl)-4-hydroxyphenyl]ethyliden]-bisphosphonsäuretetraethylester und [3,5-bis(1,1-dimethylethyl-4-hydroxyphenyl]ethenyliden]bis-phosphonsäuretetrakis(1-methylethyl)ester.

Erfindungsgemäß bevorzugte kosmetische Produkte sind dadurch gekennzeichnet, dass der mindestens eine haarwuchshemmende Wirkstoff ausgewählt ist aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, weiterhin Wirkstoffkombinationen aus Extrakten aus Epilobium Angustifolium, den Samen von Cucurbita pepo und den Früchten von Serenoa serrulata, weiterhin Wirkstoffkombinationen aus Xylitol und Extrakten von Citrus Medica Limonum (Lemon) Fruit, Carica Papaya (Papaya) und Olivenblättern, weiterhin Wirkstoffkombinationen aus Humulus Lupulus, Viscum Album, Salvia Officinalis, Carica Papaya und Thuya Occidentalis, sowie Extrakten aus Larrea divaricata.

Die W/O-Emulsionen des erfindungsgemäßen Produktes enthalten mindestens eine den Haarwuchs inhibierende Substanz, vorzugsweise in einer Menge von 0,1 - 10 Gew.-%, bevorzugt 0,5 - 5 Gew.-% und besonders bevorzugt1 - 4 Gew.-%, jeweils bezogen auf das Gewicht des Rohstoffs tel quel und das Gesamtgewicht der treibgasfreien W/O-Emulsion.

Weitere evorzugte erfindungsgemäße Zusammensetzungen enthalten mindestens eine den Haarwuchs inhibierende Substanz in einer Gesamtmenge von 0,0001 - 5 Gew.-%, bevorzugt 0,001 - 2 Gew.-%, besonders bevorzugt 0,01 - 1 Gew.-%, und außerordentlich bevorzugt 0,1 - 0,5 Gew.-%, jeweils bezogen auf das Gewicht an Aktivsubstanz des/der Haarwuchs inhibierenden Wirkstoffs/e und das Gesamtgewicht der erfindungsgemäßen treibgasfreien W/O-Emulsionszusammensetzung.

Besonders bevorzugte Wirkstoffe mit haarwuchsinhibierender Wirkung sind löslich in Wasser und/oder in Methanol und/oder in Ethanol und/oder in 1-Propanol und/oder in 2-Propanol und/oder in 1-Butanol und/oder in 2-Butanol und/oder in 2-Methyl-1-Propanol und/oder in 2-Methyl-2-Propanol und/oder in Ethandiol und/oder in 1,2-Propandiol und/oder in 1,3-Propandiol und/oder 1,2-3-Propantriol und/oder einem C₂-C₉-Polyol. Besonders bevorzugte erfindungsgemäße kosmetische Produkte sind dadurch gekennzeichnet, dass mindestens ein Wirkstoff mit haarwuchshemmender Wirkung enthalten ist, der in Wasser und/oder einem einwertigen C₁-C₄-Alkohol und/oder einem C₂-C₉-Polyol bei 20°C zu mindestens 0,1g/100 g Lösung löslich ist. Bevorzugt sind Löslichkeiten in mindestens einer der genannten Substanzen von mindestens 0,5 g / 100 g Lösung, besonders bevorzugt von mindestens 1 g / 100 g Lösung und insbesondere von mindestens 2,5 g / 100 g Lösung.

Neben den vorstehend genannten Wirkstoffen enthalten die erfindungsgemäßen Produkte mindestens einen Deodorant-Wirkstoff. Deodorant-Wirkstoffe werden exemplarisch nachstehend beschrieben:

Erfindungsgemäß bevorzugte Deodorant-Wirkstoffe sind Geruchsabsorber, deodorierend wirkende Ionenaustauscher, keimhemmende Mittel, präbiotisch wirksame Komponenten sowie Enzyminhibitoren oder, besonders bevorzugt, Kombinationen der genannten Wirkstoffe.
Silicate dienen als Geruchsabsorber, die gleichzeitig auch die rheologischen Eigenschaften der erfindungsgemäßen Zusammensetzung vorteilhaft unterstützen können. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und insbesondere 1 - 5 Gew.-%, jeweils bezogen auf das Gewicht der treibgasfreien W/O-Emulsion, eingesetzt.
Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Arylalkohole wie insbesondere Phenoxyethanol, 2-Methyl-4-phenylbutan-2-ol und 2-Methyl-5-phenylpentan-1-ol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester, insbesondere Carbonsäuremonoester des Mono-, Di- und Triglycerins (insbesondere Glycerinmonolaurat, Diglycerinmonocaprinat, Diglycerinmonolaurat, Triglycerinmonolaurat und Triglycerinmonomyristat), Lantibiotika sowie Pflanzenextrakte (z. B. grüner Tee und Bestandteile des Lindenblütenöls) einsetzbar.
Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.
Zu den Enzyminhibitoren gehören Stoffe, die die für die Schweißzersetzung verantwortlichen Enzyme, insbesondere Arylsulfatase, β-Glucuronidase, Aminoacylase, die esterspaltenden Lipasen und die Lipoxigenase, hemmen, z. B. Trialkylcitronensäureester, insbesondere Triethylcitrat, oder Zinkglycinat.
Die Deodorant-Wirkstoffe können sowohl einzeln als auch in Mischungen eingesetzt werden. Besonders bevorzugt sind Phenoxyethanol, α-(2-Ethylhexyl)glycerinether, Diglycerinmonocaprinat, 2-Methyl-4-phenylbutan-2-ol, Mischungen aus Phenoxyethanol und α-(2-Ethylhexyl)glycerinether sowie Mischungen aus Arylalkoholen, insbesondere Phenoxyethanol, mit α-(2-Ethylhexyl)glycerinether und Diglycerinmonocaprinat.
Die Menge der Deodorant-Wirkstoffe in erfindungsgemäß bevorzugten kosmetischen Produkten beträgt 0,1 - 10 Gew.-%, bezogen auf das Gesamtgewicht der treibgasfreien W/O-Emulsion. Besonders bevorzugt sind 0,2 - 7 Gew.-%, insbesondere 0,3 - 5 Gew.-% und außerordentlich bevorzugt 0,4 - 1,0 Gew.-%, jeweils bezogen auf das Gesamtgewicht der treibgasfreien W/O-Emulsion, enthalten.

Als optionale Komponente können die erfindungsgemäßen W/O-Emulsionen wasserlösliche Antitranspirant-Wirkstoffe enthalten. Erfindungsgemäß einsetzbare Antitranspirant-Wirkstoffe sind die wasserlöslichen adstringierenden anorganischen und organischen Salze des Aluminiums, Zirkoniums und Zinks bzw. beliebige Mischungen dieser Salze. Geeignete Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAI(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit der Antitranspirant-Wirkstoffe eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. In einer bevorzugten Ausführungsform enthält die Zusammensetzung ein adstringierendes Aluminiumsalz, insbesondere Aluminiumchlorohydrat, das beispielsweise pulverförmig als Micro Dry^{®} von Reheis, in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls erfindungsgemäß geeignet ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders vorteilhaft sein.

Die erfindungsgemäße Zusammensetzung ist in einer Aerosol-Abgabevorrichtung enthalten, und das kosmetische Produkt umfasst mindestens ein Treibmittel.

Erfindungsgemäß geeignete Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.
Kosmetische Produkte, die das Treibmittel in einer Menge von 10 - 90 Gew.%, bevorzugt 40 - 90 Gew.% und besonders bevorzugt 50 - 80 Gew.-%, jeweils bezogen auf das Gesamtgewicht der Zubereitung, bestehend aus der W/O-Emulsion und dem Treibmittel, enthalten, sind erfindungsgemäß bevorzugt.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der Wasser-in-Öl-Emulsion.

Die erfindungsgemäßen deodorierenden Produkte weisen trotz der Wasserphase im Aerosolbehälter eine besonders hohe Korrosionsbeständigkeit auf, was einen großen Vorteil gegenüber dem Stand der Technik darstellt. Ferner weisen die erfindungsgemäß eingesetzten Wasser-in-Öl-Emulsionen eine ausgezeichnete Hautverträglichkeit, Lagerstabilität und Wirksamkeit auf. Vorteilhaft ist insbesondere, dass die versprühten Produkte sich auf der Haut durch ein angenehmes, nicht-klebriges Hautgefühl auszeichnen. Der erfindungsgemäß ausgewogene Wasser- und Ethanolgehalt erzeugt ein angenehmes Frischegefühl mit hoher Hautverträglichkeit nach der Anwendung.

In besonders bevorzugten Ausführungsformen der vorliegenden Erfindung weist der Aerosolbehälter ein Ventil auf, das besonders ausgestaltet ist.

In einer bevorzugten Ausführungsform der Erfindung weist das Ventil einen mit einem Lack oder einem polymeren Kunststoff A beschichteten Ventilkegel und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind, sind erfindungsgemäß bevorzugt.

In einer weiteren bevorzugten Ausführungsform der Erfindung weist das Ventil ein flexibles Element mit Rückstellcharakteristik und/oder einen Ventilkegel aus mindestens einem Kunststoff B, bevorzugt einem elastomeren Kunststoff, auf. Auch hier sind erfindungsgemäße kosmetische Produkte, bei denen das Ventil ein flexibles Element mit Rückstellcharakteristik und/oder einen Ventilkegel aus mindestens einem Kunststoff B aufweist, bevorzugt, wobei bevorzugte Kunststoffe B elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element mit Rückstellcharakteristik einstückig mit dem Ventilkegel ausgebildet sein und biegsame Beine aufweisen. In einer besonders bevorzugten Ausführungsform der Erfindung sind Ventilkegel und flexibles Element mit Rückstellcharakteristik ähnlich, äquivalent oder identisch wie in WO 89/08062 A1, Figur 1 und den dazugehörigen Erläuterungen dargestellt, ausgebildet. Besonders bevorzugt ist dabei der Ventiltyp Ariane M, erhältlich von der Firma Seaquist Perfect, bei dem das flexible Element mit Rückstellcharakteristik in Form von vier elastischen Beinen einstückig mit dem Ventilkegel ausgebildet ist.

Erfindungsgemäß bevorzugt ist auch eine Ventilkonstruktion gemäß US 4,471,893.
In einer weiteren bevorzugten Ausführungsform der Erfindung weist die Abgabevorrichtung ein federloses Ventil auf, wie es zum Beispiel Gegenstand der US 2003/0102328 A1 ist.

Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten W/O-Emulsionen ebenfalls innen lackiert oder beschichtet sein. Ein erfindungsgemäß bevorzugter Innenschutzlack ist ein Epoxy-Phenollack, wie er u.a. unter der Bezeichnung Hoba 7407 P erhältlich ist.

Weitere Gegenstände der vorliegenden Erfindung sind ein Verfahren zur Verringerung und/oder zur Beseitigung von Körpergeruch, bei dem die in dem erfindungsgemäßen kosmetischen Produkt enthaltene W/O-Emulsion auf die Körperoberfläche aufgebracht wird.

Sowohl für das erfindungsgemäße Verfahren als auch für die erfindungsgemäße Verwendung gilt hinsichtlich bevorzugter Ausführungsformen mutatis mutandis das zu den erfindungsgemäßen Produkten Gesagte.

In einer weiteren bevorzugten Ausführungsform der Erfindung ist das Sprühventil so konfektioniert, dass die Emulsion mit einer Sprührate von 0,05 - 0,5 g/s, bevorzugt 0,1 - 0,35 g/s, ausgestoßen wird.

In einer weiteren bevorzugten Ausführungsform der Erfindung ist das Sprühventil so konfektioniert, dass beim Versprühen aus dem Ventil ein Spraystrahl mit einem Öffnungswinkel von mindestens 25° und höchstens 65°, bevorzugt von mindestens 30° und höchstens 60°, ausgestoßen wird.

Die nachfolgenden Beispiele sollen den Gegenstand der Erfindung erläutern, ohne ihn hierauf zu beschränken.

### Beispiele:

| Bestandteil | Beispiel 1 [Gew.-%, bezogen auf Gesamtzubereitung incl. Treibgas] | Beispiel 1 [Gew.-%, bezogen auf W/O-Emulsion] |
|---|---|---|
| Dow Corning 345 Fluid | 3,936 | 24,6 |
| 1,2-Propylen-glycol | 0,96 | 6,0 |
| Abil EM 90 | 0,48 | 3,0 |
| Phenoxyethanol | 0,08 | 0,5 |
| Wasser, vollentsalzt | 6,4 | 40,0 |
| Sensiva SC 50 | 0,048 | 0,3 |
| Menthyllactat | 0,016 | 0,1 |
| Ethanol (99,9 Vol.-%, DEP vergällt) | 4,08 | 25,5 |
| n-Butan | 84,0 | - |

Die Beispielzusammensetzung 1 wurde in eine Aluminiumdose, die innen mit einem Epoxy-Phenollack beschichtet und mit dem Ventil Ariane M, erhältlich von der Firma Seaquist Perfect, und einem innen mit Microflex-Lack beschichteten Ventilteller ausgerüstet war, abgefüllt und für 12 Wochen bei 45 °C gelagert.

### Liste der verwendeten Rohstoffe

| Dow Corning 345 Fluid | Cyclomethicone (Decamethylcyclopentasiloxan, Dodecamethylcyclohexasiloxan) | Dow Corning |
|---|---|---|
| Dow Corning 200 Fluid 5 cst | Dimethicone | Dow Corning |
| Cosmacol^{®} EMI | Di-C_{12 - 13} Alkyl Malate | Nordmann Rassmann |
| Optacool A | Menthyllactat, Menthylglycolat Menthol (1:1:0,4) | Symrise |
| Abil EM 90 | CETYL PEG/PPG-10/1 DIMETHICONE | Degussa |
| Sensiva SC 50 | ETHYLHEXYLOXYGLYCERIN | Schülke & Mayr |

## Patentansprüche

1. Deodorierendes kosmetisches Produkt, umfassend
- eine Wasser-in-Öl-Emulsion mit mindestens einem Deodorant-Wirkstoff,
- mindestens ein Treibmittel und
- eine Aerosol-Abgabevorrichtung,
**dadurch gekennzeichnet, dass** weiterhin mindestens 10 Gew.-% Ethanol, bezogen auf das Gewicht der treibgasfreien W/O-Emulsion, enthalten ist.

2. Kosmetisches Produkt gemäß Anspruch 1, **dadurch gekennzeichnet, dass** die Wasser-in-Öl-Emulsion 15 - 50 Gew.-%, bevorzugt 24 - 40 Gew.-% Ethanol, bezogen auf das Gewicht der treibgasfreien W/O-Emulsion, enthält.

3. Kosmetisches Produkt gemäß einem der vorstehenden Ansprüche, **dadurch gekennzeichnet, dass** die Wasser-in-Öl-Emulsion eine Ölphase von 1 - 60 Gew.-%, bevorzugt 5 - 30 Gew.-%, besonders bevorzugt 22 - 26 Gew.-%, jeweils bezogen auf das Gesamtgewicht der treibgasfreien W/O-Emulsion, enthält.

4. Kosmetisches Produkt gemäß einem der vorstehenden Ansprüche, **dadurch gekennzeichnet, dass** die Wasser-in-Öl-Emulsion Wasser in einer Menge von 30 - 60 Gew.-%, bevorzugt 40 - 50 Gew.-%, jeweils bezogen auf das Gesamtgewicht der treibgasfreien W/O-Emulsion, enthält.

5. Kosmetisches Produkt gemäß einem der vorstehenden Ansprüche, **dadurch gekennzeichnet, dass** die Wasser-in-Öl-Emulsion mindestens einen Wasser-in-Öl-Emulgator in einer Gesamtmenge von 0,1 - 10 Gew.-%, bevorzugt 0,5 - 5 Gew.-%, besonders bevorzugt 1 - 3 Gew.-%, jeweils bezogen auf das Gesamtgewicht der treibgasfreien W/O-Emulsion, enthält.

6. Kosmetisches Produkt gemäß einem der vorstehenden Ansprüche, **dadurch gekennzeichnet, dass** mindestens ein Wirkstoff mit haarwuchshemmender Wirkung enthalten ist, der in Wasser und/oder einem einwertigen C₁-C₄-Alkohol und/oder einem C₂-C₉-Polyol bei 20°C zu mindestens 0,1g/100g löslich ist.

7. Kosmetisches Produkt gemäß einem der vorstehenden Ansprüche, **dadurch gekennzeichnet, dass** die Ölphase zu mindestens 90 Gew.-% aus bei 20 °C flüssigen Ölkomponenten besteht.

8. Kosmetisches Produkt gemäß einem der vorstehenden Ansprüche, **dadurch gekennzeichnet, dass** die Ölkomponenten ausgewählt sind aus Decamethylcyclopentasiloxan, Dodecamethylcyclohexasiloxan, Hexamethyldisiloxan (L₂), Octamethyltrisiloxan (L₃), Decamethyltetrasiloxan (L₄), beliebigen Mischungen, insbesondere Zweier- und Dreiermischungen, aus L₂, L₃ und/oder L₄, weiterhin 2-Ethylhexylpalmitat, Hexyldecyllaurat, 2-Ethylhexylstearat, 2-Ethylhexyllaurat, Isopropylmyristat, Isopropylpalmitat, C₁₂-C₁₅-Alkylbenzoat, C₁₂-C₁₅-Alkyllactat, Di-C₁₂-C₁₃-Alkylmalat, PPG-14-Butylether, Isododecan, Isohexadecan, Isoeicosan, Polyisobuten und Polydecene sowie Mischungen der genannten Komponenten.

9. Kosmetisches Produkt gemäß einem der vorstehenden Ansprüche, **dadurch gekennzeichnet, dass** die Ölphase eine Ölmischung, die zu mehr als 50 Gew.-% mindestens ein flüchtiges cyclisches oder lineares Siliconöl enthält, umfasst.

10. Kosmetisches Produkt gemäß einem der vorstehenden Ansprüche, **dadurch gekennzeichnet, dass** mindestens 80 Gew.-% der Ölkomponenten einen Brechungsindex von 1,39 - 1,49 bei 20 °C aufweisen.

11. Kosmetisches Produkt gemäß einem der vorstehenden Ansprüche, **dadurch gekennzeichnet, dass** mindestens ein wasserlösliches mehrwertiges C₂ - C₉-Alkanol mit 2 - 6 Hydroxylgruppen und/oder wasserlösliches Polyethylenglycol mit 3 - 20 Ethylenoxid-Einheiten in einer Gesamtmenge von 0,5 - 15 Gew.-%, bevorzugt 4 - 10 Gew.-% und besonders bevorzugt 5 - 7 Gew.-%, jeweils bezogen auf die gesamte treibgasfreie W/O-Emulsion, enthalten ist.

12. Kosmetisches Produkt gemäß einem der vorstehenden Ansprüche, **dadurch gekennzeichnet, dass** mindestens ein W/O-Emulgator, ausgewählt aus Cetyl PEG/PPG 10/1 Dimethicone , enthalten ist.

13. Kosmetisches Produkt gemäß einem der vorstehenden Ansprüche, **dadurch gekennzeichnet, dass** das Treibmittel in einer Menge von 10 - 90 Gew.%, bevorzugt 40 - 90 Gew.% und besonders bevorzugt 50 - 80 Gew.-%, jeweils bezogen auf das Gesamtgewicht der Zubereitung, bestehend aus der W/O-Emulsion und dem Treibmittel, enthalten ist.

14. Kosmetisches Produkt gemäß einem der vorstehenden Ansprüche, **dadurch gekennzeichnet, dass** die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind.

15. Kosmetisches Produkt gemäß Anspruch 15, **dadurch gekennzeichnet, dass** das flexible Element mit Rückstellcharakteristik als Spiralfeder oder Schraubendruckfeder ausgebildet ist.

16. Kosmetisches Produkt gemäß Anspruch 16, **dadurch gekennzeichnet, dass** das flexible Element mit Rückstellcharakteristik einstückig mit dem Ventilkegel gefertigt ist und biegsame Beine aufweist.

17. Kosmetisches Produkt gemäß einem der Ansprüche 15 - 17, **dadurch gekennzeichnet, dass** das Ventil ein flexibles Element mit Rückstellcharakteristik und/oder einen Ventilkegel aus mindestens einem Kunststoff B aufweist.

18. Kosmetisches Produkt gemäß Anspruch 18, **dadurch gekennzeichnet, dass** der Kunststoff B ein elastomerer Kunststoff ist.

19. Kosmetisches Produkt gemäß einem der Ansprüche 1 bis 14, **dadurch gekennzeichnet, dass** die Abgabevorrichtung ein federloses Ventil aufweist.

20. Kosmetisches Produkt gemäß einem der Ansprüche 1 - 20, **dadurch gekennzeichnet, dass** das Ventil einen innenlackierten Ventilteller aufweist, wobei Lackierung und Ventilmaterial miteinander kompatibel sind.

21. Kosmetisches Produkt gemäß einem der Ansprüche 1 - 21, **dadurch gekennzeichnet, dass** die Emulsion mit einer Sprührate von 0,05 - 0,5 g/s, bevorzugt 0,1 - 0,35 g/s, ausgestoßen wird.

22. Kosmetisches Produkt gemäß einem der Ansprüche 1 - 22, **dadurch gekennzeichnet, dass** beim Versprühen aus dem Ventil ein Spraystrahl mit einem Öffnungswinkel von mindestens 25° und höchstens 65°, bevorzugt von mindestens 30° und höchstens 60°, ausgestoßen wird.

23. Verfahren zur Verringerung und/oder zur Beseitigung von Körpergeruch, **dadurch gekennzeichnet, dass** die in dem kosmetischen Produkt gemäß einem der Ansprüche 1 bis 23 enthaltene W/O-Emulsion auf die Körperoberfläche aufgebracht wird.
